(19) Europäisches Patentamt
European Patent Office
Office européen des brevets

(11) Veröffentlichungsnummer: **0 008 071**
**B1**

(12) **EUROPÄISCHE PATENTSCHRIFT**

(45) Veröffentlichungstag der Patentschrift: **20.01.82**

(51) Int. Cl.³: **C 07 D 233/22,** C 07 D 401/12, C 07 D 417/12, A 01 N 43/78, A 01 N 43/50

(21) Anmeldenummer: **79102751.9**

(22) Anmeldetag: **01.08.79**

(54) **1-Iminomethylen-substituierte 2-(Phenoxy-alkyl)-2-imidazolinderivate, Verfahren zu ihrer Herstellung, Mittel, welche diese Derivate als aktive Komponente enthalten und ihre Verwendung zur Bekämpfung von Schädlingen.**

(30) Priorität: **14.08.78 CH 8631/78**
**08.03.79 CH 2250/79**
**03.07.79 CH 6194/79**

(43) Veröffentlichungstag der Anmeldung: **20.02.80 Patentblatt 80/4**

(45) Bekanntmachung des Hinweises auf die Patenterteilung: **20.01.82 Patentblatt 82/3**

(84) Benannte Vertragsstaaten: **AT BE CH DE FR GB IT NL**

(56) Entgegenhaltungen:
**DE-A-1 935 479**
**DE-A-2 818 367**
**FR-M-3 005**

(73) Patentinhaber: **CIBA-GEIGY AG, Patentabteilung Postfach, CH-4002 Basel (CH)**

(72) Erfinder: **Böger, Manfred, Lindenstrasse 33, D-7858 Weil am Rhein 5 (DE)**
Erfinder: **Drabek, Jozef, Dr., Benkenstrasse 12, CH-4104 Oberwil (CH)**
Erfinder: **Mattern, Günter, Dr., Sichternstrasse 35, CH-4410 Liestal (CH)**
Erfinder: **Traber, Walter, Dr., Neueneichweg 12, CH-4153 Reinach (CH)**

**EP 0 008 071 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1-Iminomethylen-substituierte 2-(Phenoxy-alkyl)-2-imidazolinderivate, Verfahren zu ihrer Herstellung, Mittel, welche diese Derivate als aktive Komponente enthalten und ihre Verwendung zur Bekämpfung von Schädlingen

Die vorliegende Erfindung betrifft neue 1-Iminomethylen-substituierte 2-(Phenoxy-alkyl)-2-imidazolinderivate, welche eine Wirkung gegen Schädlinge besitzen, und Verfahren zu ihrer Herstellung sowie Schädlingsbekämpfungsmittel, welche die vorstehend genannten Derivate als aktive Komponente enthalten, und Verfahren zur Bekämpfung von Schädlingen unter Verwendung der neuen Verbindungen.

2-(Phenoxy-alkyl)-2-imidazolinderivate mit pestizider, vor allem ektoparasitizider Wirkung sind bekannt (siehe z.B. südafrikanische Patentanmeldung Nr. 78/2449, japanische Patentveröffentlichung 76/106 739, DOS 2 756 638 und DOS 2 756 639). Nach vorliegender Erfindung werden neuartige Verbindungen dieses Typs bereitgestellt, die ebenfalls eine Wirkung gegen Schädlinge, insbesondere gegen Vertreter der Ordnung Akarina aufweisen und welche aufgrund ihrer vorteilhaften biologischen Eigenschaften für die praktische Anwendung besonders geeignet sind.

Die erfindungsgemässen, neuen 1-Iminomethylen-substituierten 2-(Phenoxy-alkyl)-2-imidazolinderivate entsprechen der Formel I

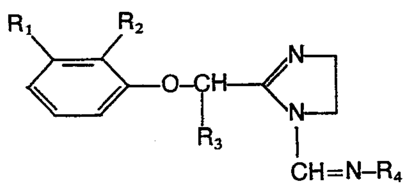

(I)

worin

$R_1$ und $R_2$ unabhängig voneinander ein Chloratom oder eine Methylgruppe,

$R_3$ ein Wasserstoffatom oder eine $C_1$–$C_4$-Alkylgruppe und

$R_4$ eine Cyangruppe oder eine Gruppe der Formel A oder B

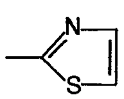

(A)

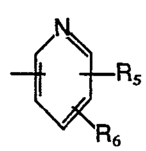

(B)

worin $R_5$ und $R_6$ unabhängig voneinander für ein Wasserstoffatom oder eine Methylgruppe stehen, bedeuten.

Die für $R_3$ in Frage kommenden Alkylgruppen sind die Methyl-, Äthyl-, n-Propyl-, i-Propyl, n-Butyl-, i-Butyl-, sek-Butyl- und t-Butylgruppe.

In den Verbindungen der Formel I werden die folgenden Substituententypen sowie Kombinationen derselben untereinander bevorzugt:

1) Bei $R_1$ und $R_2$: gleichzeitig Methyl oder gleichzeitig Chlor; 2) Bei $R_3$: Wasserstoff oder Äthyl, insbesondere Äthyl;

3) Bei $R_4$: Eine Gruppe der Formel B, insbesondere eine Gruppe der Formel B′

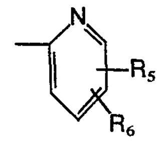

(B′)

worin $R_5$ und $R_6$ je Wasserstoff oder Methyl bedeuten.

Die Verbindungen der Formel I liegen auch in Form von Säureadditionssalzen, z.B. Mineral-Salzen, vor und können erfindungsgemäss in Form ihrer Salze verwendet werden. Unter dem Begriff der vorliegenden Erfindung versteht man demzufolge sowohl die freien Verbindungen der Formel I als auch deren Säureadditionssalze.

Es hat sich nun überraschenderweise gezeigt, dass die erfindungsgemässen Verbindungen der Formel I eine wertvolle Wirkung sowohl gegen pflanzenschädigende Akariden (Milben: z.B. der Familien Tetranychidae, Tarsonemidae, Eriophyidae, Tyroglyphidae und Glycyphagidae) als auch gegen ektoparasitäre Akariden (Milben und Zekken: z.B. der Familien Ixodidae, Argasidae, Sarciptidae und Dermanyssidae), welche an Nutztieren Schaden anrichten, aufweisen. Darüber hinaus wird nun festgestellt, dass diese akariziden Eigenschaften mit einer für die praktische Anwendung günstigen Toxizität gegenüber Warmblütern gekoppelt sind, wobei sich die Verbindungen der Formel I sowie deren für Warmblüter nichttoxische Säureadditionssalze zur Bekämpfung von Schädlingen der Ordnung Akarina in Kulturen von Nutz- und Zierpflanzen sowie zur Bekämpfung von ektoparasitären Zecken und Milben bei Nutztieren besonders eignen.

Die Verbindungen der Formel I werden analog bekannten Verfahren hergestellt, indem man z.B. eine Verbindung der Formel II

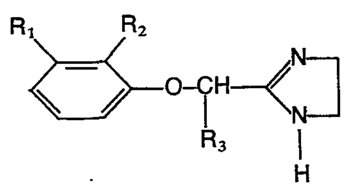

(II)

mit einer Verbindung der Formel III

$$R_7\text{–O–CH=N–}R_4 \qquad \text{(III)}$$

umsetzt, wobei in den Formeln II und III $R_1$, $R_2$, $R_3$ und $R_4$ die unter Formel I bereits angegebenen Bedeutungen haben und $R_7$ für eine Methyl- oder Äthylgruppe steht.

Das Verfahren wird zweckmässig bei einer Temperatur zwischen −20 und 120 °C, bei normalem oder leicht erhöhtem Druck und vorzugsweise in Gegenwart eines gegenüber den Reaktionsteilnehmern inerten Lösungs- oder Verdünnungsmittels durchgeführt. Als Lösungs- oder Verdünnungsmittel eignen sich z.B. Äther und

ätherartige Verbindungen wie Di-äthyläther, Di-isopropyläther, Dioxan und Tetrahydrofuran; aromatische Kohlenwasserstoffe wie Benzol, Toluol und Xylole; und Keton wie Aceton, Methyläthylketon und Cyclohexanon.

Die auf diese Weise hergestellten Verbindungen der Formel I können nach an sich bekannten Methoden in ihre Säuresalze überführt werden.

Die Verbindungen der Formel I, worin $R_3$ eine Alkylgruppe bedeutet, liegen in Form von optisch aktiven Isomeren vor. Wenn deshalb bei der Herstellung keine optisch aktiven Ausgangsmaterialien verwendet werden, gelangt man zwangsläufig zu racemischen Gemischen. Solche Isomerengemische können z.B. mit Hilfe chromatographischer Trennmethoden in die einzelnen isomeren Formen aufgetrennt werden. Unter dem Begriff der gegenwärtigen Erfindung versteht man sowohl die einzelnen optisch aktiven Isomere als auch deren Gemische.

Die in dem vorstehend aufgeführten Verfahren verwendeten Ausgangsstoffe sind bekannt (vgl. südafrikanische Patentanmeldung Nr. 78/2449 und DOS 2 756 638) bzw. können analog den bekannten Methoden hergestellt werden.

Die Verbindungen der Formel I werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger- und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die akarizide Wirkung der erfindungsgemässen Mittel lässt sich durch Zusatz anderer Akarizide und/oder Insektizide wesentlich verbreitern.

Als Zusätze eignen sich z.B.: org. Phosphorverbindungen; Nitrophenole und deren Derivate; Formamidine; Harnstoffe; pyrethrinartige Verbindungen; Karbamate und chlorierte Kohlenwasserstoffe.

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Verbindung der Formel I) in den oben beschriebenen Mitteln liegt zwischen 0,1 bis 95%, dabei ist zu erwähnen, dass bei der Applikation aus dem Flugzeug oder mittels anderer geeigneter Applikationsgeräte auch höhere Konzentrationen eingesetzt werden können.

Die Wirkstoffe der Formel I können beispielsweise wie folgt formuliert werden:

Emulsionskonzentrat I

20 Gew.-Teile des obengenannten Wirkstoffes werden in
70 Gew.-Teilen Xylol gelöst und mit
10 Gew.-Teilen eines Emulgiermittels, bestehend aus einem Gemisch eines Arylphenylpolyglykoläthers und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

Emulsionskonzentrat II

5 bis max. 30 Gew.-Teile Wirkstoff werden unter Rühren bei Zimmertemperatur in
30 Gew.-Teilen Dibutylphthalat
10 Gew.-Teilen Solvent 200 (niederviskoses hocharomat. Erdöldestillat)
15 bis 35 Gew.-Teilen Dutrex 238 FC (viskoses hocharomat. Erdöldestillat) gelöst und mit
10 Gew.-Teilen eines Emulgatorgemisches, bestehend aus Ricinusölpolyglykoläther und dem Calciumsalz der Dodecylbenzolsulfonsäure, versetzt. Das so erhaltene Emulsionskonzentrat gibt in Wasser milchige Emulsionen.

Spritzpulver

5 bis 30 Gew.-Teile des Wirkstoffes werden in einer Mischapparatur mit
5 Gew.-Teilen eines aufsaugenden Trägermaterials (Kieselsäure K 320 oder Wessalon S) und
55 bis 80 Gew.-Teilen eines Trägermaterials (Bolus alba oder Kaolin B 24) und einem Dispergiermittelgemisch, bestehend aus
5 Gew.-Teilen eines Na-lauryl-sulfonates und
5 Gew.-Teilen eines Alkyl-aryl-polyglykoläthers, intensiv vermischt. Diese Mischung wird auf einer Stift- oder Lufstrahlmühle bis auf 5–15 $\mu$m gemahlen. Das so erhaltene Spritzpulver gibt in Wasser eine gute Suspension.

Stäubemittel

5 Gew.-Teile feingemahlener Wirkstoff werden mit
2 Gew.-Teilen einer gefällten Kieselsäure und
93 Gew.-Teilen Talk intensiv gemischt.

Pour-on-Lösung

| | |
|---|---|
| Wirkstoffsubstanz | 30,0 g |
| Natrium-dioctylsulfosuccinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natrium-dioctylsulfosuccinat und das Erdnussöl gegeben und unter Erwärmen und gründlichem Durchmischen gelöst.

Die nachfolgenden Beispiele dienen zur näheren Erläuterung der Erfindung.

Beispiel 1

Herstellung von
1-[N-(2-Pyridyl)formimino]-2-[1-(2,3-dimethylphenoxy)-propyl]-2-imidazolin

Zu einer Lösung von 11,6 g
2-[1-(2,3-Dimethylphenoxy)-propyl]-2-imidazolin der Formel

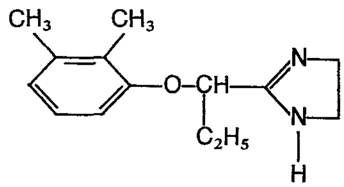

in 100 ml Toluol wurde 7,5 g N-(2-Pyridyl)-formiminoäthyläther der Formel

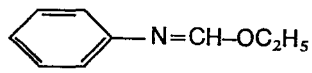

zugegeben und das Reaktionsgemisch während 12 Stunden bei 70 °C gerührt. Anschliessend wurde die erhaltene Lösung eingedampft und der Rückstand aus Toluol/Hexan umkristallisiert. Auf diese Weise erhielt man das 1-[N-(2-Pyridyl)-formimino]-2-[1-(2,3-dimethylphenoxy)-propyl]-2-imidazolin der Formel

(Verbindung Nr. 1)

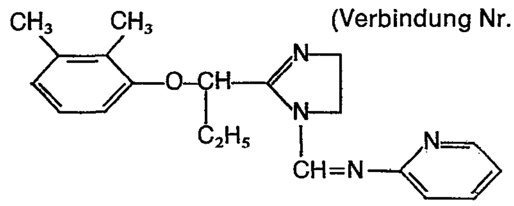

als weisses Pulver mit einem Smp. von 108–110 °C.

Analog zu dem vorstehend beschriebenen Herstellungs-Verfahren wurden auch die folgenden Verbindungen der Formel I hergestellt:

2.

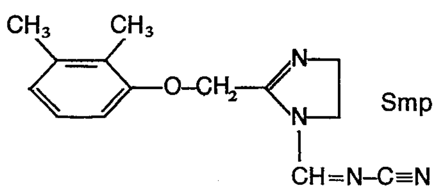

Smp. 172–173 °C

3.

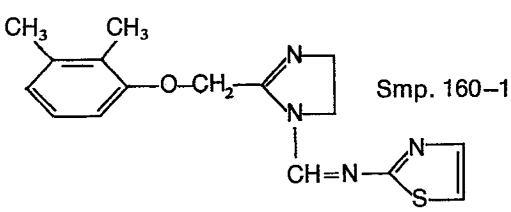

Smp. 160–162 °C

4.

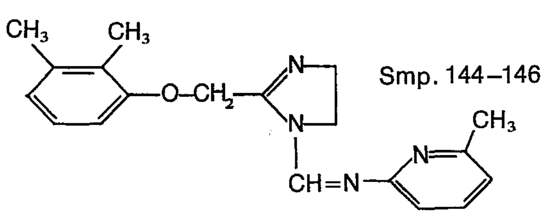

Smp. 144–146 °C

5.

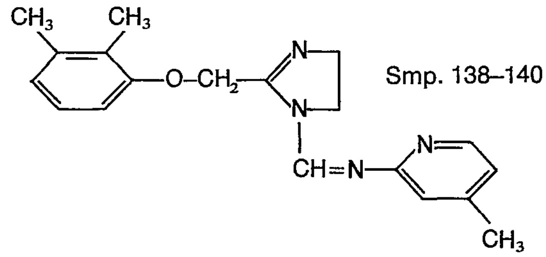

Smp. 138–140 °C

6.

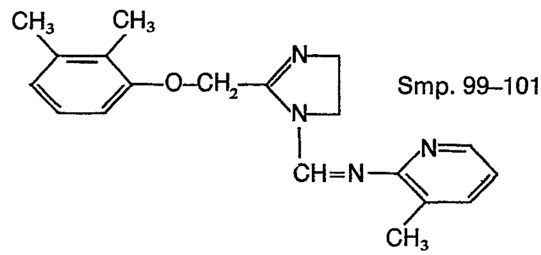

Smp. 99–101 °C

7.

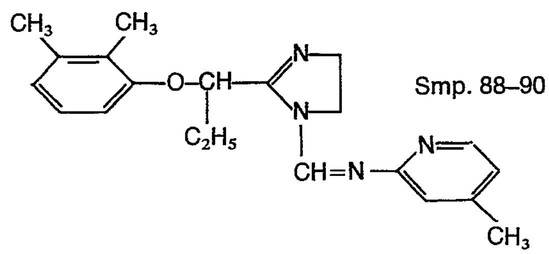

Smp. 88–90 °C

8.

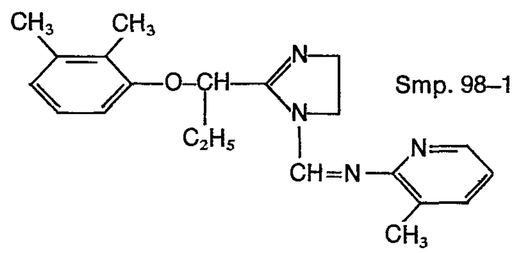

Smp. 98–100 °C

9.

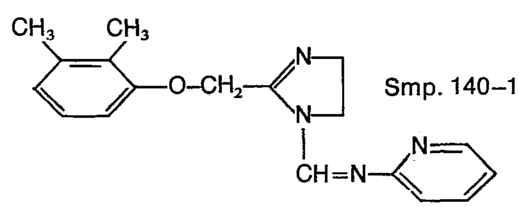

Smp. 140–142 °C

10.

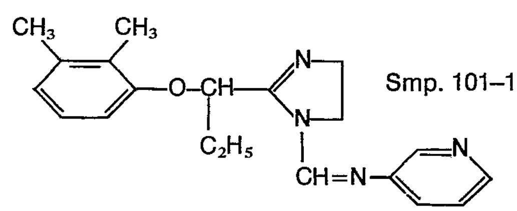

Smp. 101–102 °C

11.

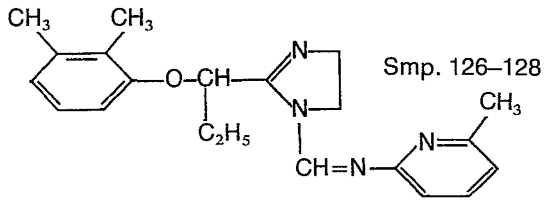

Smp. 126–128 °C

12.

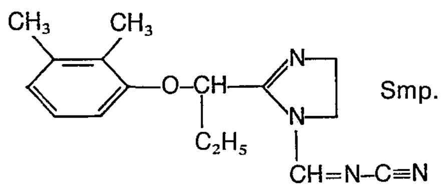

Smp. 130–132 °C

13.

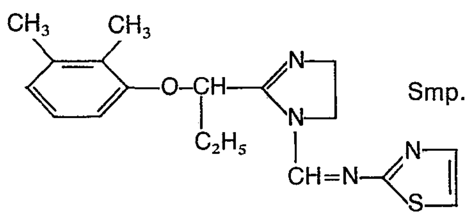

Smp. 78–80 °C

14.

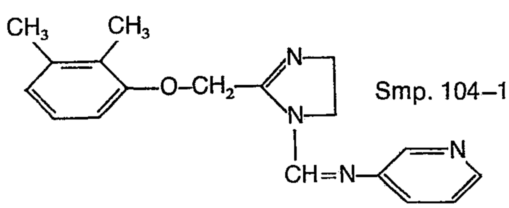

Smp. 104–106 °C

15.

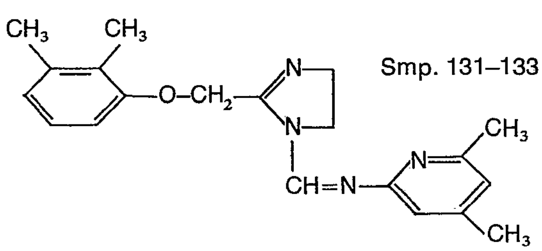

Smp. 131–133 °C

16.

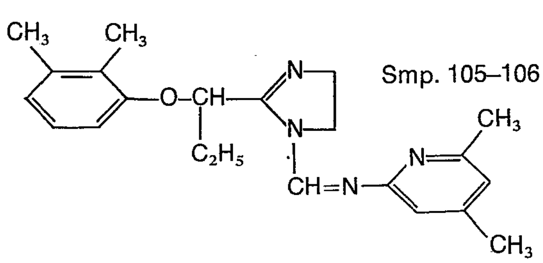

Smp. 105–106 °C

17.

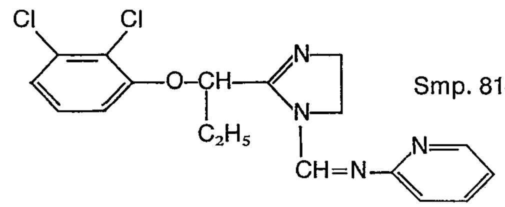

Smp. 81–83 °C

18.

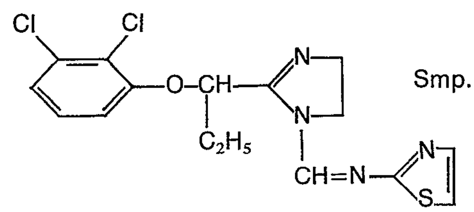

Smp. 96–98 °C

19.

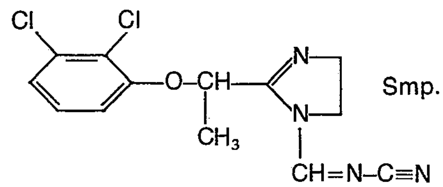

Smp. 130–132 °C

20.

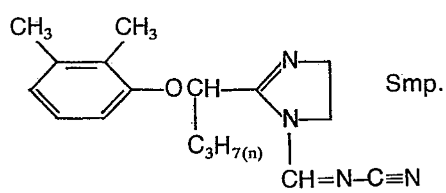

Smp. 130–131 °C

Beispiel 2

Wirkung gegen pflanzenschädigende Akariden: Tetranychus urticae (OP-sensible) und Tetranychus cinnabarinus (OP-tolerant)

Die Primärblätter von Phaseolus vulgaris-Pflanzen wurden 16 Stunden vor dem Versuch auf akarizide Wirkung mit einem infestierten Blattstück aus einer Massenzucht von Tetranychus urticae (OP-sensible) oder Tetranychus cinnabarinus (OP-tolerant) belegt. (Die Toleranz bezieht sich auf die Verträglichkeit von Diazinon.)

Die so behandelten infestierten Pflanzen wurden mit einer Versuchslösung enthaltend 400 oder 200 ppm der zu prüfenden Verbindung bis zur Tropfnässe besprüht.

Nach 24 Stunden und wiederum nach 7 Tagen wurden Imagines und Larven (alle beweglichen Stadien) unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25 °C.

Verbindungen gemäss Beispiel 1 zeigen in diesem Versuch eine positive Wirkung gegen Individuen der Spezies Tetranychus urticae und Tetranychus cinnabarinus.

Beispiel 3

Wirkung gegen ektoparasitäre Akariden: (Zecken) Rhipicephalus bursa (Imagines und Larven), Amblyomma hebraeum (♀ Imagines, Nymphen und Larven) und Boophilus microplus (Larven – OP-sensible und OP-tolerant)

Als Testobjekte werden Larven (jeweils etwa 50), Nymphen (jeweils etwa 25) oder Imagines (jeweils etwa 10) der Zeckenarten Rhipicephalus bursa, Amblyomma hebraeum und Boophilus microplus verwendet. Die Testtiere werden für kurze Zeit in eine wässrige Emulsion bzw. Lösung enthaltend 0,1; 1,0; 10; 50 oder 100 ppm der zu prüfenden Verbindung getaucht.

Die in Teströhrchen befindlichen Emulsionen bzw. Lösungen werden dann mit Watte aufgenommen und die benetzten Testtiere in den so kontaminierten Röhrchen belassen.

Eine Auswertung der erzielten Abtötungsrate bei jeder Konzentration erfolgte für Larven nach 3 Tagen und für Nymphen und Imagines nach 14 Tagen.

Verbindungen gemäss Beispiel 1 zeigen in diesem Versuch eine gute Wirkung gegen Larven, Nymphen und Imagines der Spezies Rhipicaphalus bursa und Amblyomma hebraeum sowie gegen Larven (OP-res. und OP-sens.) der Spezies Boophilus microplus.

**Patentansprüche**

für Vertragsstaaten: BE, CH, DE, FR, GB, IT und NL

1. Eine Verbindung der Formel I

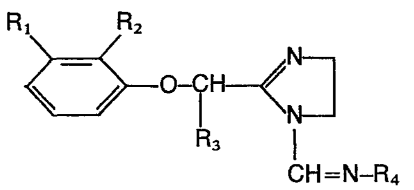

(I)

worin

$R_1$ und $R_2$ unabhängig voneinander ein Chloratom oder eine Methylgruppe,

$R_3$ ein Wasserstoffatom oder eine $C_1$–$C_4$-Alkylgruppe und

$R_4$ eine Cyangruppe oder eine Gruppe der Formel A oder B

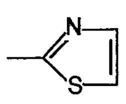

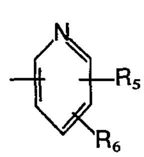

(A) (B)

worin $R_5$ und $R_6$ un~~abhängig~~ ~~voneinander~~ für ein Wasserstoffatom oder eine Methylgruppe stehen, bedeuten, und deren Säureadditionssalze.

2. Verbindung nach Anspruch 1 der Formel I, worin $R_1$ und $R_2$ gleichzeitig eine Methylgruppe oder gleichzeitig ein Chloratom bedeuten.

3. Verbindung nach Anspruch 1 oder 2 der Formel I, worin $R_3$ ein Wasserstoffatom oder eine Äthylgruppe bedeutet.

4. Verbindung nach Anspruch 3 der Formel I, worin $R_3$ eine Äthylgruppe bedeutet.

5. Verbindung nach einem der Ansprüche 1 bis 4, worin $R_4$ eine Gruppe der Formel B′

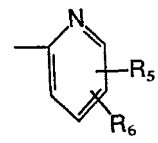

(B′)

worin $R_5$ und $R_6$ die im Anspruch 1 bereits angegebenen Bedeutungen haben bedeutet.

6. Verbindung nach Anspruch 1 oder 5, worin $R_6$ ein Wasserstoffatom bedeutet.

7. Verbindung nach Anspruch 6 der Formel

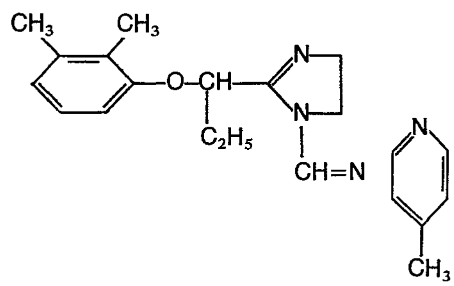

8. Verbindung nach Anspruch 6 der Formel

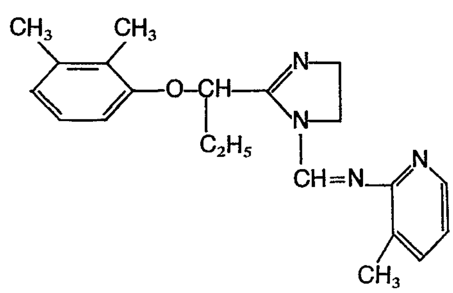

9. Verfahren zur Herstellung von in einem der Ansprüche 1 bis 8 definierten Verbindungen, dadurch gekennzeichnet, dass man eine Verbindung der Formel II

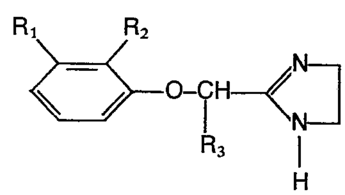

(II)

mit einer Verbindung der Formel III

$$R_7\text{–O–CH=N–}R_4 \qquad \text{(III)}$$

umsetzt, worin $R_1$, $R_2$, $R_3$ und $R_4$ die entsprechenden, in einem der Ansprüche 1 bis 8 angegebenen Bedeutungen haben und $R_7$ eine Methyl- oder Äthylgruppe bedeutet.

10. Schädlingsbekämpfungsmittel enthaltend als aktive Komponente eine der in einem der Ansprüche 1 bis 8 definierten Verbindungen.

11. Verwendung von einer der in einem der Ansprüche 1 bis 8 definierten Verbindungen zur Bekämpfung von Vertretern der Ordnung Akarina.

**Patentansprüche**
für Vertragsstaat: AT

1. Akarizides Mittel enthaltend als aktive Komponente eine Verbindung der Formel I

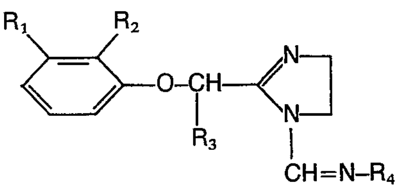

(I)

worin $R_1$ und $R_2$ unabhängig voneinander ein Chloratom oder eine Methylgruppe, $R_3$ ein Wasserstoffatom oder eine $C_1$–$C_4$-Alkylgruppe und $R_4$ eine Cyangruppe oder eine Gruppe der Formel A oder B

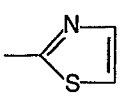

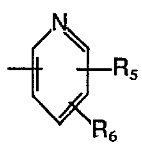

(A) (B)

in welcher $R_5$ und $R_6$ unabhängig voneinander ein Wasserstoffatom oder eine Methylgruppe darstellen, bedeuten, oder eines ihrer Säureadditionssalze.

2. Akarizides Mittel nach Anspruch 1 enthaltend als aktive Komponente eine Verbindung der Formel I, worin $R_1$ und $R_2$ gleichzeitig ein Chloratom oder gleichzeitig eine Methylgruppe bedeuten.

3. Akarizides Mittel nach Anspruch 1 oder 2 enthaltend als aktive Komponente eine Verbindung der Formel I, worin $R_3$ ein Wasserstoffatom oder eine Äthylgruppe bedeutet.

4. Akarizides Mittel nach Anspruch 3 enthaltend als aktive Komponente eine Verbindung der Formel I, worin $R_3$ eine Äthylgruppe bedeutet.

5. Akarizides Mittel nach einem der Ansprüche 1 bis 4 enthaltend als aktive Komponente eine Verbindung der Formel I, worin $R_4$ eine Gruppe der Formel B'

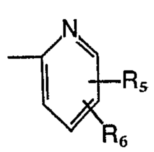

(B')

bedeutet.

6. Akarizides Mittel nach Anspruch 1 oder 5 enthaltend als aktive Komponente eine Verbindung der Formel I, worin $R_6$ ein Wasserstoffatom bedeutet.

7. Akarizides Mittel nach Anspruch 6 enthaltend als aktive Komponente eine Verbindung der Formel

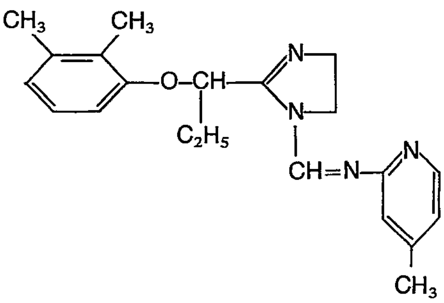

8. Akarizides Mittel nach Anspruch 6 enthaltend als aktive Komponente eine Verbindung der Formel

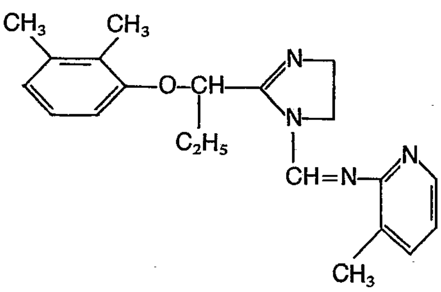

9. Verfahren zur Herstellung von in einem der Ansprüche 1 bis 8 definierten Verbindungen, dadurch gekennzeichnet, dass man eine Verbindung der Formel II

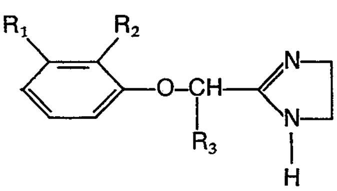

(II)

mit einer Verbindung der Formel III

$$R_7\text{–O–CH=N–}R_4 \qquad (III)$$

umsetzt, worin $R_1$, $R_2$, $R_3$ und $R_4$ die entsprechenden, in einem der Ansprüche 1 bis 8 angegebenen Bedeutungen haben und $R_7$ eine Methyl- oder Äthylgruppe bedeutet.

10. Verwendung von einer der in einem der Ansprüche 1 bis 8 definierten Verbindungen zur Bekämpfung von Vertretern der Ordnung Akarina.

**Claims**
for the Contracting States: BE, CH, DE, FR, GB, IT, NL

1. A compound of the formula I

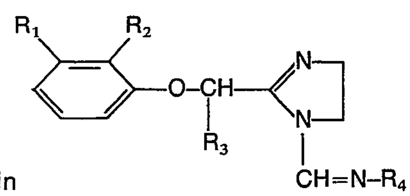

(I)

wherein

$R_1$ and $R_2$ independently of one another are each a chlorine atom or a methyl group,
$R_3$ is a hydrogen atom or a $C_1$–$C_4$-alkyl group, and
$R_4$ is a cyano group, or a group of the formula A or B

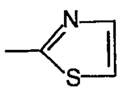

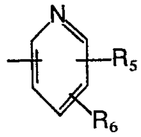

(A) (B)

wherein
$R_5$ and $R_6$ independently of one another are each a hydrogen atom or a methyl group,
and the acid addition salts thereof.

2. A compound according to Claim 1 of the formula I wherein $R_1$ and $R_2$ are either both a methyl group or both a chlorin atom.

3. A compound according to Claim 1 or 2 of the formula I wherein $R_3$ is a hydrogen atom or an ethyl group.

4. A compound according to Claim 3 of the formula I wherein $R_3$ is an ethyl group.

5. A compound according to any one of Claims 1 to 4, wherein $R_4$ is a group of the formula B′

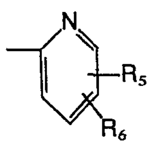

(B′)

wherein $R_5$ and $R_6$ have the meanings defined in Claim 1.

6. A compound according to Claim 1 or 5, wherein $R_6$ is a hydrogen atom.

7. A compound according to Claim 6 of the formula

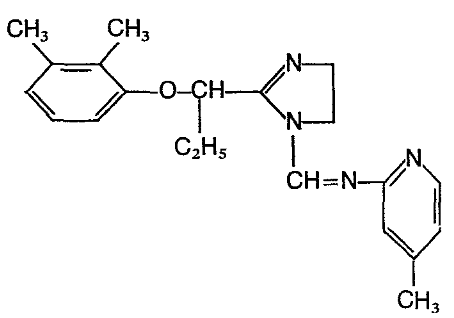

8. A compound according to Claim 6 of the formula

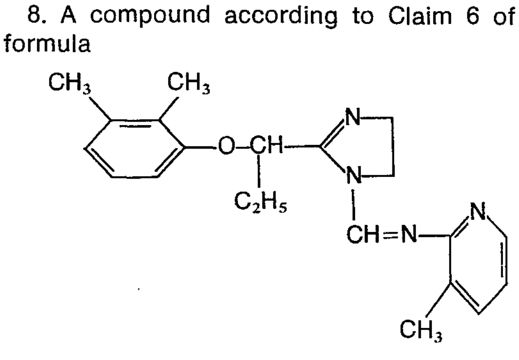

9. A process for producing a compound as defined in any one of Claims 1 to 8, which process comprises reacting a compound of the formula II

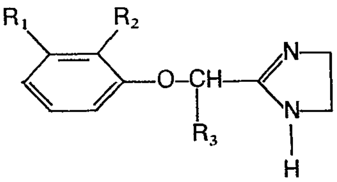

(II)

with a compound of the formula III

$$R_7\text{–O–CH=N–}R_4 \quad \text{(III)}$$

wherein $R_1$, $R_2$, $R_3$ and $R_4$ have the corresponding meanings given in any one of Claims 1 to 8, and $R_7$ is a methyl or ethyl group.

10. A pesticidal composition containing as active ingredient a compound as defined in any one of Claims 1 to 8.

11. A method of controlling representatives of the order Acarina at a locus, which method comprises applying to said locus a compound as claimed in any one of Claims 1 to 8.

**Claims**
for the Contracting State: AT

1. An acaricidal composition containing as active ingredient a compound of the formula I

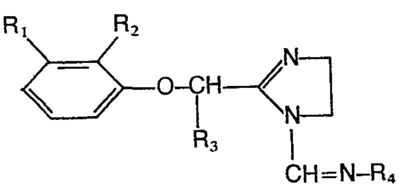

(I)

wherein
$R_1$ und $R_2$ independently of one another are each a chlorine atom or a methyl group,
$R_3$ is a hydrogen atom or a $C_1$–$C_4$-alkyl group, and
$R_4$ is a cyano group, or a group of the formula A or B

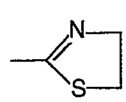

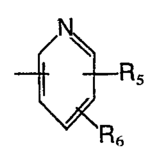

(A) (B)

wherein
$R_5$ and $R_6$ independently of one another are each a hydrogen atom or a methyl group,
or an acid addition salt thereof.

2. An acaricidal composition according to Claim 1, which contains as active ingredient a compound of the formula I wherein $R_1$ and $R_2$ are either both a chlorine atom or both a methyl group.

3. An acaricidal composition according to Claim 1 or 2, which contains as active ingredient

a compound of the formula I wherein $R_3$ is a hydrogen atom or an ethyl group.

4. An acaricidal composition according to Claim 3, which contains as active ingredient a compound of the formula I wherein $R_3$ is an ethyl group.

5. An acaricidal composition according to any one of Claims 1 to 4, which contains as active ingredient a compound of the formula I wherein $R_4$ is a group of the formula B′

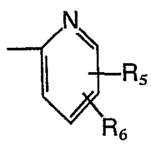

(B′)

6. An acaricidal composition according to Claum 1 or 5, which contains as active ingredient a compound of the formula I wherein $R_6$ is a hydrogen atom.

7. An acaricidal composition according to Claim 6, which contains as active ingredient a compound of the formula

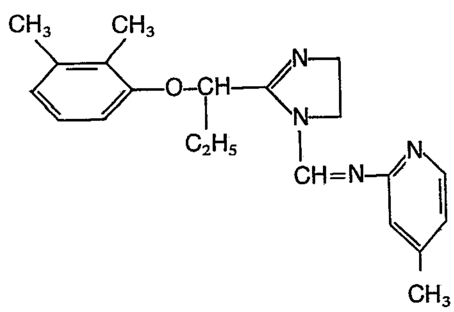

8. An acaricidal composition according to Claim 6, which contains as active ingredient a compound of the formula

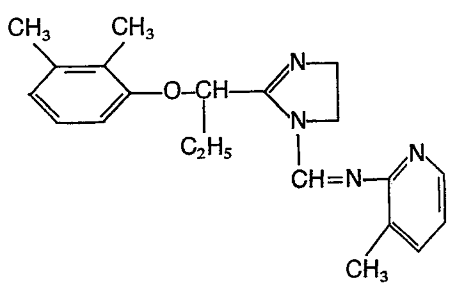

9. A process for producing a compound as defined in any one of Claims 1 to 8, which process comprises reacting a compound of the formula II

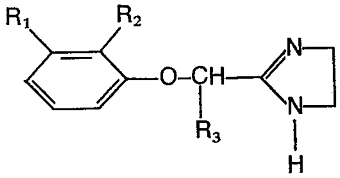

(II)

with a compound of the formula III

$$R_7\text{–O–CH=N–}R_4 \qquad \text{(III)}$$

wherein $R_1$, $R_2$, $R_3$ and $R_4$ have the corresponding meanings given in any one of Claims 1 to 8, and $R_7$ is a methyl or ethyl group.

10. A method of controlling representatives of the order Acarina at a locus, which method comprises applying to said locus a compound as claimed in any one of Claims 1 to 8.

**Revendications**

pour les Etats contractants: BE, CH, DE, FR, GB, IT, NL

1. Un composé de formule I

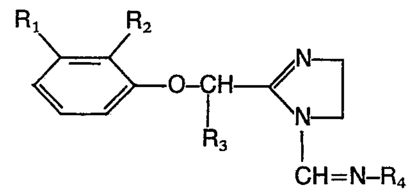

(I)

dans laquelle

$R_1$ et $R_2$ représentent chacun, indépendamment l'un de l'autre, un atome de chlore ou un groupe méthyle,

$R_3$ représente un atome d'hydrogène ou un groupe alkyle en $C_1$–$C_4$ et

$R_4$ représente un groupe cyano ou un groupe de formule A ou B

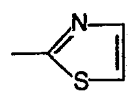

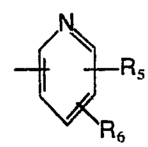

(A) (B)

dans lesquelles $R_5$ et $R_6$ représentent chacun, indépendamment l'un de l'autre, un atome d'hydrogène ou un groupe méthyle, et leurs sels formés par addition avec des acides.

2. Composé selon la revendication 1 de formule I dans laquelle $R_1$ et $R_2$ représentent tous deux un groupe méthyle ou tous deux un atome de chlore.

3. Composé selon la revendication 1 ou 2, de formule I dans laquelle $R_3$ représente un atome d'hydrogène ou un groupe éthyle.

4. Composé selon la revendication 3, de formule I dans laquelle $R_3$ représente un groupe éthyle.

5. Composé selon l'une des revendications 1 à 4, dans lequel $R_4$ représente un groupe de formule B′

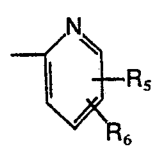

(B′)

dans laquelle $R_5$ et $R_6$ ont les significations déjà indiquées dans la revendication 1.

6. Composé selon la revendication 1 ou 5, dans lequel $R_6$ représente un atome d'hydrogène.

7. Composé selon la revendication 6, de formule

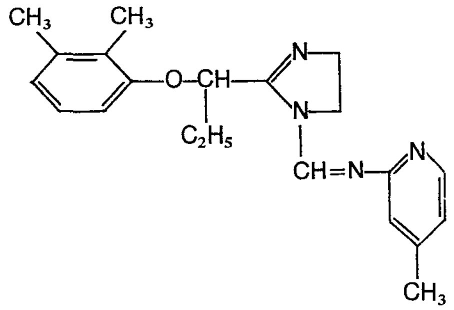

8. Composé selon la revendication 6, de formule

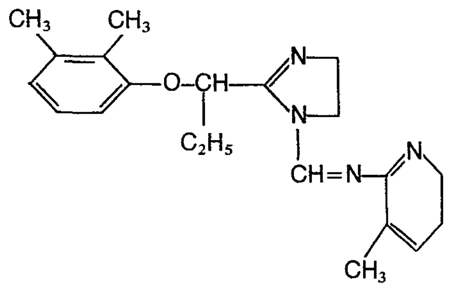

9. Procédé de préparation des composés définis dans l'une des revendications 1 à 8, caractérisé en ce que l'on fait réagir un composé de formule II

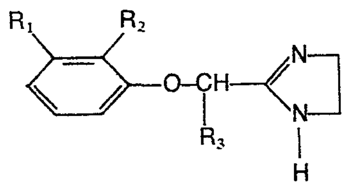
(II)

avec un composé de formule III

$$R_7\text{–O–CH=N–}R_4 \qquad \text{(III)}$$

$R_1$, $R_2$, $R_3$ et $R_4$ ayant les significations correspondantes, indiquées dans l'une des revendications 1 à 8 et $R_7$ représentant un groupe méthyle ou éthyle.

10. Produits pesticides contenant en tant que composant actif l'un des composés définis dans l'une des revendications 1 à 8.

11. Utilisation de l'un des composés définis dans l'une des revendications 1 à 8 dans la lutte contre les représentants de l'ordre des acariens.

**Revendications**
pour l'Etat contractant: AT

1. Produit acaricide contenant en tant que composant actif un composé de formule I

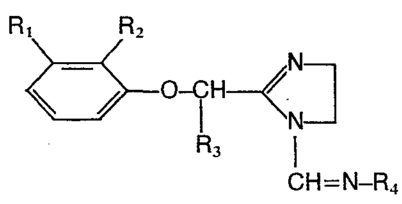
(I)

dans laquelle $R_1$ et $R_2$ représentent chacun, indépendamment l'un de l'autre, un atome de chlore ou un groupe méthyle, $R_3$ représente un atome d'hydrogène ou un groupe alkyle en $C_1$–$C_4$ et $R_4$ représente un groupe cyano ou un groupe de formule A ou B

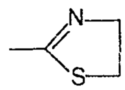
(A)

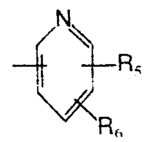
(B)

dans lesquelles $R_5$ et $R_6$ représentent chacun, indépendamment l'une de l'autre, un atome d'hydrogène ou un groupe méthyle, ou l'un de leurs sels formés par addition avec acides.

2. Produit acaticide selon la revendication 1 contenant en tant que composant actif un composé de formule I dans laquelle $R_1$ et $R_2$ représentent tous deux un atome de chlore ou tous deux un groupe méthyle.

3. Produit acaricide selon la revendication 1 ou 2, contenant en tant que composant actif un composé de formule I dans laquelle $R_3$ représente un atome d'hydrogène ou un groupe éthyle.

4. Produit acaricide selon la revendication 3, contenant en tant que composant actif un composé de formule I dans laquelle $R_3$ représente un groupe éthyle.

5. Produit acaricide selon l'une des revendications 1 à 4, contenant en tant que composant actif un composé de formule I dans laquelle $R_4$ représente un groupe de formule B'

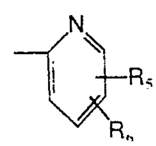
(B')

6. Produits acaricide selon la revendication 1 ou 5, contenant en tant que composant actif un composé de formule I dans laquelle $R_6$ représente un atome d'hydrogène.

7. Produit acaricide selon la revendication 6, contenant en tant que composant actif un composé de formule

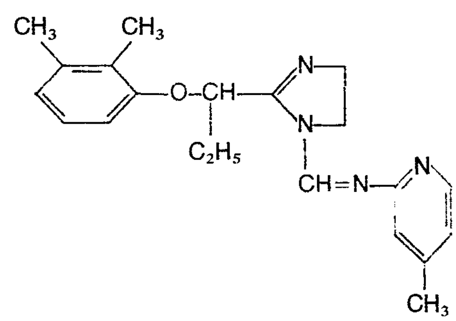

8. Produit acaricide selon la revendication 6, contenant en tant que composant actif un composé de formule

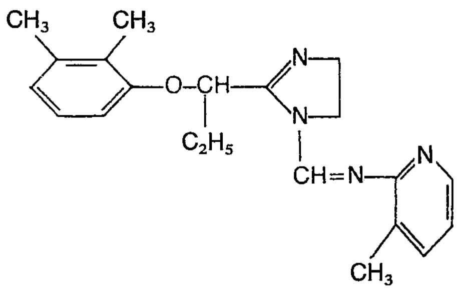

9. Procédé de préparation des composés définis dans l'une des revendications 1 à 8, caractérisé en ce que l'on fait réagir un composé de formule II

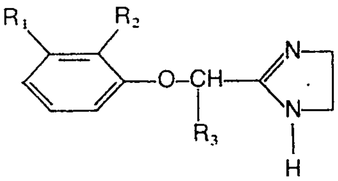

(II)

avec un composé de formule III

$$R_7-O-CH=N-R_4 \qquad (III)$$

$R_1$, $R_2$, $R_3$ et $R_4$ ayant les significations correspondantes, indiquées dans l'une des revendications 1 à 8, et $R_7$ représentant un groupe méthyle ou éthyle.

10. Utilisation de l'un des composés définis dans l'une des revendications 1 à 8 dans la lutte contre les représentants de l'ordre des acariens.